# EUROPEAN PATENT SPECIFICATION

(11) **EP 3 075 378 B1**
(45) Date of publication and mention of the grant of the patent: **01.04.2020**
(21) Application number: 14866003.8
(22) Date of filing: 20.11.2014
(51) Int. Cl.: A61K 31/357, A61K 8/34, A61K 8/49, A61Q 19/02, A61P 17/00, A61P 29/00, A61P 43/00

(54) **ANTI-INFLAMMATORY AGENT AND MELANIN PRODUCTION SUPPRESSANT**
ENTZÜNDUNGSHEMMER UND MELANINPRODUKTIONSUNTERDRÜCKER
AGENT ANTI-INFLAMMATOIRE ET SUPPRESSEUR DE LA PRODUCTION DE MÉLANINE

(30) Priority: 29.11.2013 JP 2013247208; 23.04.2014 JP 2014088864
(43) Date of publication of application: 05.10.2016
(73) Proprietor: Kao Corporation, Chuo-ku Tokyo 103-8210 (JP)
(72) Inventor: ENDOU, Youko, Odawara-shi Kanagawa 250-0002 (JP); SUGITA, Jun, Odawara-shi Kanagawa 250-0002 (JP); HARYUU, Yasushi, Odawara-shi Kanagawa 250-0002 (JP)
(74) Representative: Hoffmann Eitle
(86) International application number: PCT/JP2014/080712
(87) International publication number: WO 2015/080010

(56) References cited:
- EP-A1- 1 872 764
- WO-A1-2006/115190
- FR-A1- 2 815 253
- GB-A- 2 362 825
- JP-A- H0 648 914
- JP-A- 2012 131 748
- JP-A- 2013 249 285
- JP-A- 2014 084 304
- JP-A- 2014 084 305
- MINTEL GNPD: 'Sensual Body Lotion' ID#1464872 December 2010, XP008183676 Retrieved from the Internet: <URL:http://www.gnpd.com> [retrieved on 2015-02-14]

## Description

### Field of the Invention

The present invention relates to a skin treatment composition for suppressing PGE₂ production or melanin production and a non-medical use of the composition.

### Background of the Invention

The skin is variously affected when exposed to ultraviolet rays. In this regard, various chemical mediators produced in the skin cause inflammation, and serious damages to the skin tissue, leading to symptoms such as rough skin or pigmentation, a blotch and a freckle. In addition, typical chemical mediators include prostaglandins and leukotrienes, both of which are arachidonic acid metabolites; interleukins and tumor necrosis factor-α, which are a kind of cytokine, for example. Particularly prostaglandin E₂ (PGE₂) is widely known as a typical inflammatory mediator.

For the purpose of preventing inflammation caused by ultraviolet rays, and the like and improving its symptoms, proposed are, for example, anti-inflammatory agents such as L-α-glycero-phospho-D-myo-inositol (Patent Literature 1), Liliaceae Hemerocallis plant and a fermented product thereof (Patent Literature 2). A skin external-preparation comprising them is placed on the market.

Meanwhile, a blotch or freckle occurs from collapse of the balance of production and excretion of melanin, and is an excessive accumulation of melanin on the epidermis. As their causes, there are various causes such as inflammation, hormone balance, a genetic factor, and they are promoted by the influence of ultraviolet rays. What alleviates increased pigmentation is a skin whitening agent. Among skin whitening agents, for example, kojic acid and ascorbic acid derivatives are known, which are formulated in skin whitening cosmetics for preventing skin blackening, or a blotch or freckle, and maintaining original white skin. However, these were not always satisfactory in view of the degree of skin whitening effect and stability in the formulation.

Pentaerythritol is a kind of sugar alcohol, and is industrially used as a raw material for a rosin ester, a raw material for an alkyd resin or a raw material for a synthetic lubricant, for example. A liquid cosmetics comprising pentaerythritol has been proposed (Patent Literature 3) so far, and use of pentaerythritol as a sub-solvent or a moisturizing agent is known.
Patent Literature 1: JP 2009-269851 A
Patent Literature 2: JP 2008-50326 A
Patent Literature 3: JP H6-48914 A

### Summary of Invention

The present invention provides a skin treatment composition, as active ingredient, comprising one or more kinds selected from the group consisting of pentaerythritol, an alkyl acetal derivative of pentaerythritol represented by the formula (1) described below, and an alkyl acetal derivative of pentaerythritol represented by the formula (2) described below, for use in a method of suppressing PGE₂ production to obtain anti-inflammatory effect or suppressing melanin production: 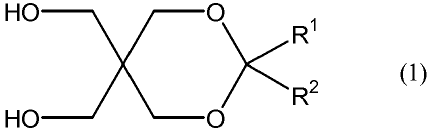 wherein R¹ and R² may be different from each other to represent C₁₋₄ alkyl; and 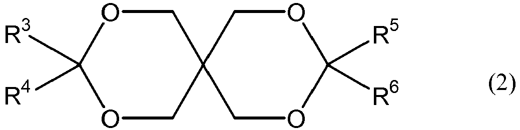 wherein R³ to R⁶ may be different from each other to represent C₁₋₄ alkyl.

In addition, the present invention provides a non-medical use of a skin external composition comprising one or more kinds selected from the group consisting of pentaerythritol, an alkyl acetal derivative of pentaerythritol represented by the formula (1) described above, and an alkyl acetal derivative of pentaerythritol represented by the formula (2) described above for suppressing melanin production.

### Detailed Description of the Invention

The anti-inflammatory agents described above may be problematic in view of safety and formulation stability when they are applied to the skin for a long time. Currently there is no anti-inflammatory agent that is satisfactory enough in each aspect of safety, formulation stability and anti-inflammation effect. In addition, a further excellent melanin production suppressant is desired.

Accordingly, the present invention provides a skin external-preparation composition having actions of suppressing inflammation due to stimulation such as ultraviolet rays, preventing rough skin, pigmentation, a blotch, or a freckle due to, for example, ultraviolet rays, and suppressing melanin production.

The present inventors conducted earnest investigation considering the situations described above, and as a result thereof, found that pentaerythritol and an alkyl acetal derivative of pentaerythritol have excellent suppressive effect for increase of PGE₂ production in epidermal cell due to stimulation such as ultraviolet rays, and further have excellent suppressive effect for melanin production. In addition, the present inventors found that a skin external-preparation composition comprising them suppresses inflammation caused by ultraviolet rays, and exerts an effect of preventing or improving rough skin, an effect of preventing pigmentation, a blotch, or a freckle, and further an effect of suppressing melanin production and an effect of whitening the skin, and completed the present invention.

Pentaerythritol and an alkyl acetal derivative of pentaerythritol of the present invention are excellent anti-inflammatory agents having excellent suppressive ability for PGE₂ production, and excellent skin whitening agents having excellent suppressive action for melanin production. Accordingly, a skin external-preparation composition comprising pentaerythritol and an alkyl acetal derivative of pentaerythritol is excellent in an effect of preventing or improving rough skin, or pigmentation, a blotch or a freckle caused by ultraviolet rays, an effect of whitening the skin, or an effect of improving the skin tone.

Hereinafter, the constitutions of the present invention will be explained in detail.

The anti-inflammatory agent, the PGE₂ production suppressant, the melanin production suppressant, or the skin whitening agent of the present invention, and a skin external-preparation composition for anti-inflammation, for preventing rough skin, for preventing sunburn, for whitening the skin or for improving the skin tone (hereinafter, also referred to as the composition of the present invention) contain one or more kinds selected from the group consisting of pentaerythritol, and an alkyl acetal derivative of pentaerythritol.

Pentaerythritol is a kind of sugar alcohol represented by a formula of C(CH₂OH)₄.

The alkyl acetal derivative of pentaerythritol is represented by the formula (1) or (2) described below: 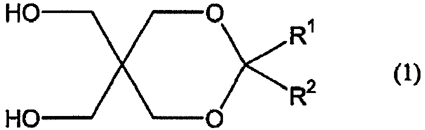 wherein R¹ and R² may be different from each other to represent C₁₋₄ alkyl; and 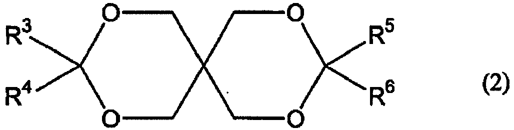 wherein R³ to R⁶ may be different from each other to represent C₁₋₄ alkyl.

The alkyl represented by R¹ to R⁶ in the formula (1) or (2) described above, is preferably C₁₋₃ alkyl, and more preferably C₁ or C₂ alkyl. Specifically, examples of the alkyl include methyl, ethyl, n-propyl and isopropyl, and further the alkyl is preferably methyl, ethyl or n-propyl, and is more preferably methyl or ethyl.

Examples of the alkyl acetal derivative of pentaerythritol represented by the formula (1) include monoisopropylidene pentaerythritol, mono(1-methylpropylidene) pentaerythritol, mono(1-methylbutylidene) pentaerythritol, mono(1-ethylpropylidene) pentaerythritol, mono(1-ethylbutylidene) pentaerythritol, and mono(1-propylbutylidene) pentaerythritol.

Examples of the alkyl acetal derivative of pentaerythritol represented by the formula (2) include diisopropylidene pentaerythritol, di(1-methylpropylidene) pentaerythritol, di(1-methylbutylidene) pentaerythritol, di(1-ethylpropylidene) pentaerythritol, di(1-ethylbutylidene) pentaerythritol, and di(1-propylbutylidene) pentaerythritol.

The alkyl acetal derivative of pentaerythritol represented by the formula (1) or (2) according to the present invention can be synthesized from pentaerythritol and an alkyl ketone compound by a known method.

Pentaerythritol, the alkyl acetal derivative of pentaerythritol represented by the formula (1), and the alkyl acetal derivative of pentaerythritol represented by the formula (2) (hereinafter, also referred to as pentaerythritols) suppress PGE₂ production from arachidonic acid in the epidermal cell, and suppress PGE₂ production caused by ultraviolet irradiation as shown in Examples described hereafter. In addition, the pentaerythritols remarkably suppress erythema caused by ultraviolet irradiation by application to the skin. Furthermore, the pentaerythritols have excellent melanin production suppressive action and skin whitening action.

Accordingly, pentaerythritol or the alkyl acetal derivative of pentaerythritol represented by the formula (1) or (2) is useful as a PGE₂ production suppressant composition, an anti-inflammatory agent composition, a melanin production suppressant composition or a skin whitening agent composition, and further as a skin external-preparation composition for anti-inflammation, for preventing rough skin, for preventing sunburn, for whitening the skin or for improving the skin tone.

The anti-inflammation, rough skin prevention and sunburn prevention effects in the skin external-preparation composition of the present invention are mainly based on anti-inflammation effect, and further based on suppression of inflammation caused by ultraviolet rays.

Accordingly, the skin external-preparation composition of the present invention is excellent in an effect of preventing or improving inflammation caused by ultraviolet irradiation, erythema from sunburn and pigmentation, a blotch, a freckle or rough skin caused by ultraviolet irradiation.

The skin whitening effect and the skin tone improving effect in the skin external-preparation composition of the present invention are mainly based on melanin production suppressive action and skin whitening action.

Examples of the skin tone improvement include improvements of freckle, irregular color and skin brightness.

Examples of the skin external-preparation composition described above include an external-preparation composition for a drug, an external-preparation composition for a quasi-drug, and a cosmetic composition. Particularly, the skin external-preparation composition is preferably used as a cosmetic composition.

The content of the pentaerythritols in the present invention is preferably 0.001% or more by mass, more preferably 0.01% or more by mass, further preferably 0.1% or more by mass and further preferably 0.5% or more by mass, and is preferably 20% or less by mass, more preferably 10% or less by mass, further preferably 8% or less by mass, and further preferably 5% or less by mass on the basis of the total amount of the composition described above. The specific range of the content of the pentaerythritols is preferably from 0.001 to 20% by mass, more preferably from 0.01 to 15% by mass, further preferably from 0.1 to 10% by mass, further preferably from 0.1 to 8% by mass, further preferably from 0.5 to 8% by mass and further preferably from 0.5 to 5% by mass. If the content is within this range, PGE₂ production suppressive ability is sufficiently exerted, and excellent anti-inflammation effect, melanin production suppressive action or skin whitening action is obtained.

The composition comprising the pentaerythritols of the present invention can be manufactured in accordance with an ordinary method, and for example, can be applied to any form such as skincare cosmetics such as a milky lotion, a cream, a lotion, a serum, a pack and a face wash; makeup cosmetics such as a lipstick, a foundation cream, a liquid foundation and a makeup pressed powder; hair cosmetics such as a hair shampoo, a hair rinse, a hair treatment, a conditioner, a hair dye and a hairdressing; cleansing cosmetics such as a face wash, a body shampoo and soap; and further bath preparations. Of course, the form of the composition comprising the pentaerythritols of the present invention is not limited thereto. Particularly, the composition comprising the pentaerythritols of the present invention is preferably applied to cosmetics as a skin external preparation such as a toning lotion, a milky lotion, a cream and a serum.

In addition to the essential ingredients described above, the composition of the present invention may contain any ingredient as necessary that is ordinarily used such that the effects of the present invention are not impaired.

When the pentaerythritols are formulated in an external preparation for skin whitening such as skin whitening cosmetics on the basis of the melanin production suppressive action thereof, a known skin whitening agent may be suitably used in combination such as hydroquinone, arbutin, ellagic acid, kojic acid, vitamin C and a derivative thereof (for example, ascorbic acid glucoside, sodium ascorbyl phosphate, disodium ascorbyl sulfate, magnesium ascorbyl phosphate, ascorbyl isopalmitate, or ethyl ascorbate), and a biphenyl derivative (for example, melanin production suppressants such as dehydrodicreosol and 2,2'-dihydroxy-5,5'-dipropyl biphenyl), Pyracantha crenulata extract, Dioscorea composita extract, elder extract, Bergenia crassifolia extract, German chamomile extract, adenosine 5'-1-phosphoric acid and a salt thereof, a linoleic acid derivative, vitamin B3 and a derivative thereof, tranexamic acid and a tranexamic acid salt, a tranexamic acid derivative, and 4-methoxysalicilic acid and a salt thereof from the viewpoint of reinforcing or supplementing the melanin production suppressive action thereof.

In addition to the ingredients described above, suitably formulated in the composition of the present invention such that the purposes of the present invention are not impaired, may be moisturizing agents such as hyaluronic acid, polyalcohol and sugar alcohol; colored pigments such as a tar-based pigment and iron oxide; preservatives such as paraben; anionic surfactants such as a fatty acid soap and sodium cetyl sulfate; nonionic surfactants such as polyoxyethylene alkyl ether, polyoxyethylene fatty acid ester, polyoxyethylene polyalcohol fatty acid ester, polyoxyethylene hydrogenated castor oil, polyalcohol fatty acid ester and polyglycerin fatty acid ester; cationic surfactants such as a tetraalkyl ammonium salt; amphoteric surfactants such as a betaine type, a sulfobetaine type, a sulfoamino acid type and a sodium N-stearoyl-L-glutamate; natural surfactants such as lecithin and lysophosphatidylcholine; natural polymers such as gelatin, casein, starch, gum arabic, karaya gum, guar gum, Locust bean gum, tragacanth gum, quince seed, pectin, carrageenan and sodium alginate; semi-synthesized polymers such as methyl cellulose, hydroxyethyl cellulose, hydroxypropyl cellulose, sodium carboxymethyl cellulose and ethyl cellulose; synthesized polymers such as polyvinyl alcohol, polyvinyl methyl ether and a copolymer, polyvinyl pyrrolidone, sodium polyacrylate, a carboxyvinyl polymer and a polyethylene oxide polymer; a thickener such as xanthan gum; pigments such as titanium oxide; or antioxidants such as dibutylhydroxytoluene.

In regard to the embodiments described above, the present invention will be further disclosed with the embodiments below.
<1> A skin treatment composition comprising one or more kinds selected from the group consisting of pentaerythritol, an alkyl acetal derivative of pentaerythritol represented by the formula (1) described below, and an alkyl acetal derivative of pentaerythritol represented by the formula (2) described below, for use in a method of suppressing PGE₂ production or suppressing melanin production: 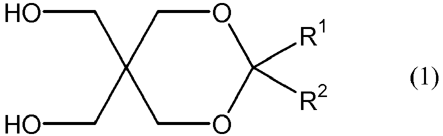 wherein R¹ and R² may be different from each other to represent C₁₋₄ alkyl; and 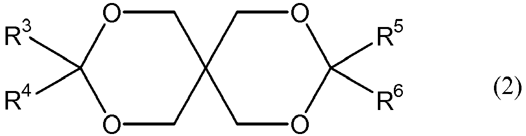 wherein R³ to R⁶ may be different from each other to represent C₁₋₄ alkyl.
<2> The composition, the use or the method of <1> described above, wherein the alkyl represented by R¹ and R² in the formula (1) is C₁₋₃ alkyl, more preferably C₁ or C₂ alkyl.
<3> The composition, the use or the method of any one of <1> to <2> described above, wherein the alkyl acetal derivative of pentaerythritol represented by the formula (1) is monoisopropylidene pentaerythritol, mono(1-methylpropylidene) pentaerythritol or mono(1-ethylpropylidene) pentaerythritol.
<4> The composition, the use or the method of any one of <1> to <3> described above, wherein the alkyl represented by R³ to R⁶ in the formula (2) is C₁₋₃ alkyl, more preferably C₁ or C₂ alkyl.
<5> The composition, the use or the method of any one of <1> to <4> described above, wherein the alkyl acetal derivative of pentaerythritol represented by the formula (2) is diisopropylidene pentaerythritol, di(1-methylpropylidene) pentaerythritol or di(1-ethylpropylidene) pentaerythritol.
<6> The composition, the use or the method of any one of <1> to <5> described above, wherein the content of pentaerythritol, or the alkyl acetal derivative of pentaerythritol is preferably 0.001% or more by mass, more preferably 0.01% or more by mass, further preferably 0.1% or more by mass and further preferably 0.5% or more by mass, and is preferably 20% or less by mass, more preferably 15% or less by mass, further preferably 10% or less by mass, further preferably 8% or less by mass and further preferably 5% or less by mass on the basis of the total amount of the composition.
<7> The composition, the use or the method of any one of <1> to <6> described above, wherein the content of pentaerythritol, or the alkyl acetal derivative of pentaerythritol is preferably from 0.001 to 20% by mass, more preferably from 0.01 to 15% by mass, further preferably from 0.1 to 10% by mass, further preferably from 0.1 to 8% by mass, further preferably from 0.5 to 8% by mass and further preferably from 0.5 to 5% by mass on the basis of the total amount of the composition.
<8> The composition, the use or the method of any one of <1> to <7> described above, wherein the active ingredient is pentaerythritol.
<9> The composition, the use or the method of any one of <1> to <8> described above, wherein the skin external-preparation composition is a cosmetic composition.

### [Examples]

Hereinafter, the present invention will be explained in detail based on Examples and Comparative Examples.

### Example 1 (Synthesis of diisopropylidene pentaerythritol)

Pentaerythritol (5.0 g, manufactured by KANTO KAGAKU) was suspended in acetone (70 mL), sulfuric acid (0.1 mL) was added, and the mixture was heated to 50°C. The mixture was reacted for a whole day and night, and then thereto was added potassium carbonate (1 g) to neutralize. About 50% of excess acetone was removed under reduced pressure. Then, ethyl acetate was added and acetone was removed. To the mixture was added water and the mixture was extracted with ethyl acetate to give diisopropylidene pentaerythritol (4.5 g) as a white crystal.

The results of ¹H NMR of diisopropylidene pentaerythritol are shown.
¹H NMR (400 MHz, CDCl₃) [δ: 1.85(s, 12H), 3.72(s, 8H)]

### Example 2 (Synthesis of monoisopropylidene pentaerythritol)

Pentaerythritol (5.0 g, manufactured by KANTO KAGAKU) was suspended in acetone (50 mL), sulfuric acid (0.05 mL) was added and the mixture was heated to 50°C. The mixture was reacted for 2 hours, and then thereto was added potassium carbonate (1 g) to neutralize, and about 50% of excess acetone was removed under reduced pressure. Then, ethyl acetate was added and acetone was removed. To the mixture was added water and the mixture was extracted with ethyl acetate to give monoisopropylidene pentaerythritol (3.0 g) as a white crystal.

The results of ¹H NMR of monoisopropylidene pentaerythritol are shown.
¹H NMR (400 MHz,CDCl₃) [δ: 1.42(s, 6H), 2.61(br, 2H), 3.73(s, 4H), 3.75(s, 4H)]

### Example 3 (Test-1 of PGE₂ production suppression)

Normal human epidermal cells were seeded on a 24 well plate by 2 × 10⁵ each, and cultured for 2 days on a MCDB153 medium. The medium was exchanged with a MCDB153 medium containing arachidonic acid (manufactured by Wako Pure Chemical Industries, Ltd.), which is a substrate of PGE₂, and pentaerythritol (manufactured by JUNSEI CHEMICAL CO., LTD.) or the two acetal derivatives of pentaerythritol (the compounds of Examples 1 and 2), and the cells were further cultured for one day. As a control, purified water was added instead of pentaerythritol or the acetal derivative of pentaerythritol. After completion of the culture, the amount of PGE₂ released in the cultured supernatant was quantified using a PGE₂ measurement kit (manufactured by Cayman Chemical Company). In addition, in the same manner as the control, a control group to which purified water was added, but arachidonic acid was not added, was provided.

The results are shown in Table 1. They show that pentaerythritol and the acetal derivative of pentaerythritol according to the present invention reduce the amount of PGE₂ increasing by addition of arachidonic acid in all of the concentration regions, and they have excellent effect to suppress the production of PGE₂.

**[Table 1]**

| | Sample concentration (µM) | PGE₂ amount (pg/mL) ± SD |
|---|---|---|
| (Control group without arachidonic acid) | - | 165.1 ± 17.42 |
| Control | - | 1711.7 ± 25.91 |
| Pentaerythritol | 0.5 | 789.2 ± 168.61 |
| | 5.0 | 1151.3 ± 6.73 |
| Monoisopropylidene pentaerythritol (Example 2) | 0.5 | 824.7 ± 28.78 |
| | 5.0 | 790.5 ± 11.64 |
| Diisopropylidene pentaerythritol (Example 1) | 0.5 | 746.0 ± 28.70 |
| | 5.0 | 1142.0 ± 19.94 |

### Example 4 (Test-2 of PGE₂ production suppression)

Normal human epidermal cells were seeded on a 24 well plate by 2 × 10⁵ each, and cultured for two days on a MCDB153 medium. The medium was replaced with HEPES Buffer, and was irradiated with 30 mJ/cm² UVB using a UVB lamp (TOREX FL20S and E-30/DMR 20W manufactured by TOSHIBA MEDICAL SUPPLY CO., LTD.). The intensity of ultraviolet rays was measured with a digital type ultraviolet intensity meter (manufactured by Ultraviolet). After the UVB irradiation, the medium was exchanged with a MCDB153 medium containing pentaerythritol (manufactured by JUNSEI CHEMICAL CO., LTD.), and further cultured for one day. As a control, purified water was added instead of pentaerythritol. After completion of the culture, the amount of PGE₂ released in the culture supernatant was quantified using a PGE₂ measurement kit (manufactured by Cayman Chemical Company). In addition, in the same manner as the control, a control group containing purified water and not irradiated with UVB was provided.

The results are shown in Table 2. They show that pentaerythritol according to the present invention reduces the amount of PGE₂ that is increased by UVB irradiation, and that pentaerythritol has excellent effect to suppress the production of PGE₂ .

**[Table 2]**

| | Sample concentration (µM) | PGE₂ amount (pg/mL) ± SD |
|---|---|---|
| (Control group not irradiated with UVB) | | 476.6 ± 14.91 |
| Control | | 670.1 ± 23.97 |
| Pentaerythritol | 0.05 | 565.5 ± 28.10 |
| | 0.5 | 522.8 ± 50.40 |
| | 5.0 | 512.3 ± 65.31 |

### Example 5 (Erythema suppression test)

A gel containing pentaerythritol of the present invention was prepared by a usual method in accordance with the composition shown in Table 3, and the erythema suppression test described below was carried out.

### Test method

Two sites on the back of three persons of the subjects were irradiated with ultraviolet ray corresponding to 1.56 MED using Solar Simulator (manufactured by Solar Light). Immediately after this ultraviolet irradiation, the gel containing the pentaerythritol was applied in 25 µL/cm² to one side of the back of the subject. Further, 3 hours after ultraviolet irradiation, 25 µL/cm² of the gel was again applied similarly to those described above. Then, 24 hours after ultraviolet irradiation, the redness was determined with determination of erythema by visual observation and the erythema index given by Mexameter MX16 (manufactured by Courage Khhazaka). The comparison of the site on which the pentaerythritol-containing formulation was applied and the non-applied site was performed by the visual observation. The results are shown in Tables 4 and 5.

**[Table 3]**

| Pentaerythritol-containing gel | |
|---|---|
| (Ingredient) | (Content: % by mass) |
| Pentaerythritol | 4.0 |
| Dipropylene glycol | 10.0 |
| Glycerin | 5.0 |
| 95% Ethyl alcohol | 5.0 |
| Methyl polysiloxane | 2.0 |
| Polyoxyethylene hydrogenated castor oil | 0.5 |
| Phenoxyethanol | 0.3 |
| Carboxyvinyl polymer | 0.3 |
| Potassium hydroxide | 0.16 |
| Disodium edetate | 0.01 |
| Purified water | 72.73 |

**[Table 4]**

| Result of erythema determination | |
|---|---|
| (Strength of redness) | (Person) |
| Formulation-applied group > Not-applied group | 0 |
| Formulation-applied group = Not-applied group | 1 |
| Formulation-applied group < Not-applied group | 2 |

**[Table 5]**

| Result of redness measurement (Measured value ± standard deviation) | | |
|---|---|---|
| | Pentaerythritol-containing formulation | Not-applied group |
| Subject 1 | 602.7 ± 5.7 | 616.3 ± 5.0 |
| Subject 2 | 635.0 ± 14.4 | 650.3 ± 7.5 |
| Subject 3 | 613.3 ± 9.0 | 622.3 ± 6.1 |

From Table 4 described above, it was judged that the redness of the group to which the pentaerythritol-containing formulation of the present invention was applied, was weak in two persons out of the three persons and judged that the redness was equal in one person in comparison to the non-applied group.

In addition, from the results of Maxameter in Table 5 described above, it is understood that any group to which the pentaerythritol-containing formulation of the present invention was applied, shows lower value in the erythema index and is excellent in erythema suppressive effect in comparison to the non-applied group. Accordingly, one can understand that the skin external-preparation and cosmetics of the present invention suppress inflammation caused by ultraviolet rays, and are excellent in the effects of preventing or improving rough skin, pigmentation, a blotch, or a freckle.

There was no subject that was recognized to have skin stimulation reaction and skin sensitization reaction on the site to which the formulation was applied during the test period, and it was confirmed that the product of the present invention is also safe in the form of a formulation.

### Example 6 (Test of melanin production suppression, using three-dimensional cultured skin model)

With respect to pentaerythritol, the test of melanin production suppression described below was conducted.

### • Test method

A three-dimensional cultured skin model (MEL-300-A, manufactured by MatTek Corporation) composed of normal human epidermal melanocytes and epidermal keratinocytes was cultured, using the attached medium to which factors inducing melanin production were added. 25 µL of a pentaerythritol solution (1 or 5 mmol/L) was added onto the keratinous layer of the skin model. The skin model was cultured for 11 days exchanging the medium and the test substance solution every 2 to 3 days, and then the skin model was washed, and the cell survival rate was measured using Alamar Blue (manufactured by Invitrogen). In addition, the skin model was dissolved under heating in SOLVABLE (manufactured by Perkin Elmar), and the absorbance (405 nm) was measured and the amount of melanin was calculated relative to a standard determined by similarly dissolving melanin (manufactured by SIGMA) in SOLVABLE. The amount of melanin was determined relative to 100 (%) of the control (H₂O addition).

The results are shown in Table 6.

**[Table 6]**

| Compound | (mmol/L) | (%) |
|---|---|---|
| Control (H₂O) | - | 100 |
| Pentaerythritol | 1 | 83.8 |
| Pentaerythritol | 5 | 76.7 |

From the results of Table 6, it was shown that pentaerythritol suppresses melanin production in a dose dependent manner, and has good skin whitening action. Meanwhile, cell toxicity was not detected in any test addition concentration described above.

### Example 7 (Test of melanin production suppression, using three dimensional cultured skin model)

Because Example 6 showed a melanin production suppressive effect by pentaerythritol, test of melanin production suppression was conducted for three kinds of sugar alcohol (erythritol, mannitol and mannose), which are analogous to each other. The test method is similar to that of Example 6.

The results are shown in Table 7.

**[Table 7]**

| Compound | Addition concentration (mmol/L) | Melanin amount (%) |
|---|---|---|
| Control (H₂O) | - | 100 |
| Erythritol | 5 | 97.0 |
| Mannitol | 5 | 96.3 |
| Mannose | 5 | 99.6 |

The results of Table 7 show that the melanin production suppressive effect of erythritol, mannitol or mannose was not recognized. Meanwhile, the cell toxicity was not detected in any test addition concentration described above.

### Example 8 (Half face comparative test in continuous use)

The half face comparative test in continuous use described below was carried out using a serum containing pentaerythritol, which is an ingredient for suppressing melanin production of the present invention, and the state of the skin after the use was evaluated.

### (Method for continuous use)

To healthy 22 female subjects ranging from 33 to 48 years old (38.1 years old on average), the serum containing the pentaerythritol (Example 8) was applied twice a day on a half face, and a placebo serum (not containing pentaerythritol; Comparative Example 1) applied to the other half face by a double blind test, and evaluation was conducted eight weeks after continuous use. The formulation of the serum is shown in Table 8.

**[Table 8]**

| (Prescription of test serum) | | |
|---|---|---|
| (Content; % by mass) | | |
| | Example 8 | Comparative Example 1 |
| Pentaerythritol | 2.0 | 0 |
| Dipropylene glycol | 10.0 | 10.0 |
| Glycerin | 5.0 | 5.0 |
| 95% Ethyl alcohol | 5.0 | 5.0 |
| Methyl polysiloxane | 2.0 | 2.0 |
| Polyoxyethylene | 0.5 | 0.5 |
| hydrogenated castor oil | | |
| Phenoxyethanol | 0.3 | 0.3 |
| Carboxyvinyl polymer | 0.3 | 0.3 |
| Potassium hydroxide | 0.16 | 0.16 |
| Disodium edetate | 0.01 | 0.01 |
| Purified water | Balance | Balance |

### (Evaluation: Questionnaire)

The subjects were asked to answer how the skin state had changed (freckle, color unevenness, brightness of the skin) on the right and the left of the skin 8 weeks after the continuous use in comparison to that before the test initiation. Five-Step evaluation (5: better, 4: somewhat better, 3: no change, 2: somewhat worse and 1: worse) was conducted with respect to each item, and subjects who answered either 5 or 4 among all the subjects were regarded as a "group who actually felt effective for the applied sample", and the ratio of the group was determined.

**[Table 9]**

| | Better freckle | Better irregular color | Better brightness of the skin |
|---|---|---|---|
| Example 8 | 18% | 27% | 50% |
| Comparative Example 1 | 14% | 23% | 41% |

As also shown from the results of Table 9, the ratio of the subjects who actually felt that freckle, irregular color and brightness of the skin on the site applied with Example 8 containing pentaerythritol of the present invention were improved in comparison to the site applied with Comparative Example 1, is greater. Thus, it is understood that Example 8 containing pentaerythritol of the present invention is more effective. In addition, there was no subject that experienced troubles on the skin, and the like and discontinued with the test the during this test period.

The skin external-preparation compositions of the present invention having the formulations described below were manufactured with a usual method (Examples 9 to 13). None of the skin external-preparation compositions was recognized to have problems in formulating properties, and the like. Meanwhile, all of the formulating amounts are represented by % by mass.

### Example 9 (Milky lotion)

**[Table 10]**

| (Ingredient) | (Content) |
|---|---|
| Diisopropylidene pentaerythritol (Example 1) | 5.0 |
| Bentonite | 0.5 |
| Glyceryl monostearate | 1.0 |
| Stearic acid | 0.5 |
| Behenyl alcohol | 0.3 |
| Cholesterol | 0.3 |
| Vaseline | 0.5 |
| Liquid paraffin | 10.0 |
| Methyl polysiloxane | 1.0 |
| Conc. glycerin | 10.0 |
| Dipropylene glycol | 10.0 |
| Sodium N-stearoyl-L-glutamate | 0.2 |
| Xanthan gum | 0.3 |
| Ascorbic acid 2-glucoside | 2.0 |
| Dipotassium glycyrrhizinate | 0.2 |
| Nicotinic acid amide | 1.0 |
| N-acetylglucosamine | 0.1 |
| N-methyl-L-serine | 0.1 |
| Phellodendron bark extract | 0.2 |
| Apricot kernel extract | 0.1 |
| Yeast extract | 0.1 |
| 1,2-octanediol | 0.2 |
| Phenoxyethanol | 0.1 |
| Purified water | Balance |

### Example 10 (Toning lotion)

**[Table 11]**

| (Ingredient) | (content) |
|---|---|
| Diisopropylidene pentaerythritol (Example 1) | 0.5 |
| Glycerin | 10.0 |
| 1,3-Butylene glycol | 6.0 |
| Ethanol | 8.0 |
| Polyoxyethylene hydrogenated castor oil (60E.O.) | 0.5 |
| Ascorbic acid | 1.0 |
| Citric acid | 0.1 |
| Sodium citrate | 0.3 |
| Purified water | Balance |

### Example 11 (Cream)

**[Table 12]**

| (Ingredient) | (Content) |
|---|---|
| Di(1-ethylpropylidene) pentaerythritol | 0.2 |
| Pentaerythritol (manufactured by KANTO KAGAKU) | 1.0 |
| Stearic acid | 5.0 |
| Stearyl alcohol | 5.0 |
| Glyceryl tri-2-ethylhexanoate | 10.0 |
| Macadamia nut oil fatty acid cholesteryl | 4.0 |
| dl-α-tocopherol acetate | 0.2 |
| Glyceryl monostearate | 3.0 |
| Dipropylene glycol | 10.0 |
| 1,2-Hexanediol | 1.0 |
| Potassium hydroxide | 0.2 |
| Phenoxyethanol | 0.3 |
| Methyl paraben | 0.1 |
| Perfume | q.s. |
| Purified water | Balance |

### Example 12 (Cream)

**[Table 13]**

| (Ingredient) | (Content) |
|---|---|
| Pentaerythritol | 2.0 |
| Olive squalane | 10.0 |
| Palmitic acid | 2.0 |
| Hydrogenated palm kernel oil | 0.3 |
| Macadamia nut oil | 0.2 |
| Meadowfoam seed oil | 0.2 |
| Jojoba oil | 0.1 |
| Apricot kernel oil | 0.1 |
| Hydrogenated soybean phospholipid | 0.2 |
| Cetanol | 3.5 |
| Glyceryl monopalmitate | 2.0 |
| Conc. glycerin | 10.0 |
| Phenoxyethanol | 0.1 |
| Carboxyvinyl polymer | 0.2 |
| Potassium hydroxide | 0.2 |
| Purified water | Balance |

### Example 13 (Sunscreen)

**[Table 14]**

| (Ingredient) | (Content) |
|---|---|
| Diisopropylidene pentaerythritol (Example 1) | 0.5 |
| Pentaerythritol (manufactured by KANTO KAGAKU) | 3.0 |
| Methyl polysiloxane (1.5 mm²/s) | 1.0 |
| Methyl polysiloxane (2.0 mm²/s) | 2.0 |
| Methyl polysiloxane (6.0 mm²/s) | 1.0 |
| Methyl phenylpolysiloxane | 2.0 |
| Methyl cyclopolysiloxane | 4.0 |
| Tris(trimethylsiloxy)methyl silane | 4.0 |
| Polyoxyethylene / methyl polysiloxane copolymer | 2.0 |
| Isododecane | 1.0 |
| Isohexadecane | 2.0 |
| Liquid isoparaffin | 2.0 |
| Isononyl isononanoate | 5.0 |
| Squalane | 1.0 |
| 2-Ethylhexyl p-methoxycinnamate | 5.0 |
| Dimethyldistearyl ammonium hectorite | 1.0 |
| Zinc oxide | 10.0 |
| Titanium oxide | 5.0 |
| 1,2-Butanediol | 1.0 |
| Phenoxyethanol | 0.3 |
| Pure water | Balance |

### Example 14 (Milky lotion)

**[Table 15]**

| (Ingredient) | (Content) |
|---|---|
| Pentaerythritol (manufactured by KANTO KAGAKU) | 5.0 |
| Bentonite | 0.5 |
| Glyceryl monostearate | 1.0 |
| Stearic acid | 0.5 |
| Behenyl alcohol | 0.3 |
| Cholesterol | 0.3 |
| Vaseline | 0.5 |
| Liquid paraffin | 10.0 |
| Methyl polysiloxane | 1.0 |
| Conc. glycerin | 10.0 |
| Dipropylene glycol | 10.0 |
| Sodium N-stearoyl-L-glutamate | 0.2 |
| Xanthan gum | 0.3 |
| Ascorbic acid 2-glucoside | 2.0 |
| Dipotassium glycyrrhizinate | 0.2 |
| Nicotinic acid amide | 1.0 |
| N-acetylglucosamine | 0.1 |
| N-methyl-L-serine | 0.1 |
| Phellodendron bark extract | 0.2 |
| Apricot kernel extract | 0.1 |
| Yeast extract | 0.1 |
| 1,2-octanediol | 0.2 |
| Phenoxyethanol | 0.1 |
| Purified water | Balance |

### Example 15 (Toning lotion)

**[Table 16]**

| (Ingredient) | (Content) |
|---|---|
| Pentaerythritol (manufactured by KANTO KAGAKU) | 0.5 |
| Glycerin | 10.0 |
| 1,3-Butylene glycol | 6.0 |
| Ethanol | 8.0 |
| Polyoxyethylene hydrogenated castor oil (60E.O.) | 0.5 |
| Ascorbic acid | 1.0 |
| Citric acid | 0.1 |
| Sodium citrate | 0.3 |
| Purified water | Balance |

### Example 16 (Cream)

**[Table 17]**

| (Ingredient) | (Content) |
|---|---|
| Pentaerythritol (manufactured by KANTO KAGAKU) | 1.0 |
| Stearic acid | 5.0 |
| Stearyl alcohol | 5.0 |
| Glyceryl tri-2-ethylhexanoate | 10.0 |
| Macadamia nut oil fatty acid cholesteryl | 4.0 |
| dl-α-tocopherol acetate | 0.2 |
| Glyceryl monostearate | 3.0 |
| Dipropylene glycol | 10.0 |
| 1,2-Hexanediol | 1.0 |
| Potassium hydroxide | 0.2 |
| Phenoxyethanol | 0.3 |
| Methyl paraben | 0.1 |
| Perfume | q.s. |
| Purified water | Balance |

### Example 18 (Sunscreen)

**[Table 18]**

| (Ingredient) | (Content) |
|---|---|
| Pentaerythritol (manufactured by KANTO KAGAKU) | 3.0 |
| Methyl polysiloxane (1.5 mm2/s) | 1.0 |
| Methyl polysiloxane (2.0 mm2/s) | 2.0 |
| Methyl polysiloxane (6.0 mm2/s) | 1.0 |
| Methyl phenylpolysiloxane | 2.0 |
| Methyl cyclopolysiloxane | 4.0 |
| Tris(trimethylsiloxy)methyl silane | 4.0 |
| Polyoxyethylene / methyl polysiloxane copolymer | 2.0 |
| Isododecane | 1.0 |
| Isohexadecane | 2.0 |
| Liquid isoparaffin | 2.0 |
| Isononyl isononanoate | 5.0 |
| Squalane | 1.0 |
| 2-Ethylhexyl p-methoxycinnamate | 5.0 |
| Dimethyldistearyl ammonium hectorite | 1.0 |
| Zinc oxide | 10.0 |
| Titanium oxide | 5.0 |
| 1,2-Butanediol | 1.0 |
| Phenoxyethanol | 0.3 |
| Pure water | Balance |

### Industrial Applicability

The anti-inflammatory agent, the melanin production suppressant and the skin whitening agent comprisng pentaerythritol and an alkyl acetal derivative of pentaerythritol of the present invention have excellent prostaglandin E₂ production suppressive effect, erythema suppressive effect, melanin production suppressive effect and skin whitening effect, and are excellent in safety and stability, and can be stably formulated in a skin external-preparation composition for the purpose of anti-inflammation, prevention of rough skin, prevention of sunburn, skin whitening, skin tone improvement, or the like.

## Claims

1. A skin treatment composition comprising one or more compounds, as active ingredient, selected from the group consisting of pentaerythritol, an alkyl acetal derivative of pentaerythritol represented by the formula (1) described below, and an alkyl acetal derivative of pentaerythritol represented by the formula (2) described below, for use in a method of suppressing PGE₂ production to obtain anti-inflammatory effect or suppressing melanin production: 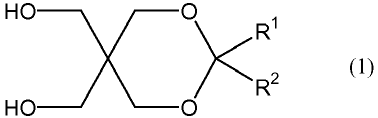 wherein R¹ and R² may be different from each other to represent C₁₋₄ alkyl; and 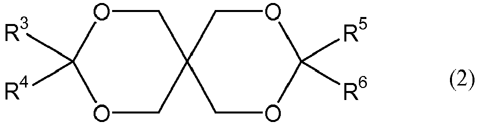 wherein R³ to R⁶ may be different from each other to represent C₁₋₄ alkyl.

2. The composition according to claim 1, wherein the active ingredient is pentaerythritol.

3. The composition according to claim 1 or 2, wherein the alkyl represented by R¹ and R² in the formula (1) is C₁ or C₂.

4. The composition according to claim 1 or 2, wherein the alkyl represented by R³ to R⁶ in the formula (2) is C₁ or C₂.

5. The composition according to any one of claims 1 to 4, wherein the content of the one or more compounds selected from the group consisting of pentaerythritol, the alkyl acetal derivative of pentaerythritol represented by the formula (1), and the alkyl acetal derivative of pentaerythritol represented by the formula (2), is from 0.001 to 15% by mass on the basis of the total amount of the composition.

6. Non-medical use of a skin external-preparation composition comprising one or more compounds selected from the group consisting of pentaerythritol, an alkyl acetal derivative of pentaerythritol represented by the formula (1) described below, and the alkyl acetal derivative of pentaerythritol represented by the formula (2) described below, for suppressing melanin production: 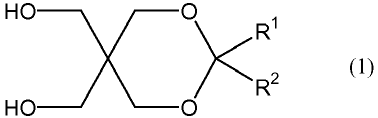 wherein R¹ and R² may be different from each other to represent C₁₋₄ alkyl; and 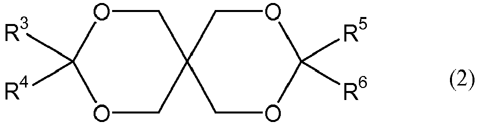 wherein R³ to R⁶ may be different from each other to represent C₁₋₄ alkyl.

7. The non-medical use according to claim 6, wherein the active ingredient is pentaerythritol.

8. The non-medical use according to claim 6 or 7, wherein the alkyl represented by R¹ and R² in the formula (1) is C₁ or C₂.

9. The non-medical use according to claim 6 or 7, wherein the alkyl represented by R³ to R⁶ in the formula (2) is C₁ or C₂.

10. The non-medical use according to any one of claims 6 to 9, wherein the content of the one or more compounds selected from the group consisting of pentaerythritol, the alkyl acetal derivative of pentaerythritol represented by the formula (1), and the alkyl acetal derivative of pentaerythritol represented by the formula (2), is from 0.001 to 15% by mass on the basis of the total amount of the composition.

## Patentansprüche

1. Hautbehandlungszusammensetzung, enthaltend eine oder mehrere Verbindungen als aktiven Bestandteil, ausgewählt aus der Gruppe, bestehend aus Pentaerythrit, einem Alkylacetalderivat von Pentaerythrit, dargestellt durch die Formel (1), die unten beschrieben ist, und einem Alkylacetalderivat von Pentaerythrit, dargestellt durch die Formel (2), die unten beschrieben ist, zur Verwendung in einem Verfahren zum Unterdrücken der PGE₂-Produktion, unter Erhalt einer Antientzündungswirkung oder Unterdrückung der Melaninproduktion: 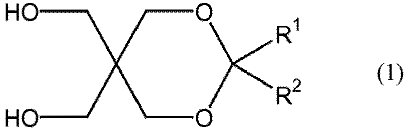 worin R¹ und R² gleich oder verschieden voneinander sein können und C₁₋₄-Alkyl sind, und 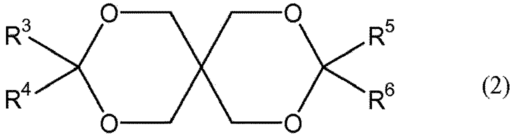 worin R³ bis R⁶ gleich oder verschieden voneinander sein können und C₁₋₄-Alkyl sind.

2. Zusammensetzung gemäß Anspruch 1, worin der aktive Bestandteil Pentaerythrit ist.

3. Zusammensetzung gemäß Anspruch 1 oder 2, worin das Akyl, dargestellt durch R¹ und R² in der Formel (1), C₁ oder C₂ ist.

4. Zusammensetzung gemäß Anspruch 1 oder 2, worin das Akyl, dargestellt durch R³ bis R⁶ in der Formel (2), C₁ oder C₂ ist.

5. Zusammensetzung gemäß einem der Ansprüche 1 bis 4, worin der Gehalt der einen oder mehreren Verbindungen, ausgewählt aus der Gruppe, bestehend aus Pentaerythrit, dem Alkylacetalderivat von Pentaerythrit mit der Formel (1) und dem Alkylacetalderivat von Pentaerythrit mit der Formel (2) von 0,001 bis 15 Masse-% auf der Basis der Gesamtmenge der Zusammensetzung ist.

6. Nicht-medizinische Verwendung einer externen Hauptpräparatzusammensetzung, enthaltend eine oder mehrere Verbindungen, ausgewählt aus der Gruppe, bestehend aus Pentaerythrit, einem Alkylacetalderivat von Pentaerythrit mit der unten beschriebenen Formel (1) und dem Alkylacetalderivat von Pentaerythrit mit der unten beschriebenen Formel (2), zur Unterdrückung der Melaninerzeugung: 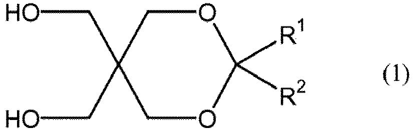 worin R¹ und R² gleich oder verschieden voneinander sein können und C₁₋₄-Alkyl sind, und 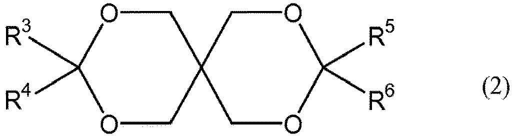 worin R³ bis R⁶ gleich oder verschieden voneinander sein können und C₁₋₄-Alkyl sind.

7. Nicht-medizinische Verwendung gemäß Anspruch 6, worin der aktive Bestandteil Pentaerythrit ist.

8. Nicht-medizinische Verwendung gemäß Anspruch 6 oder 7, worin das Alkyl, dargestellt durch R¹ und R² in der Formel (1), C₁ oder C₂ ist.

9. Nicht-medizinische Verwendung gemäß Anspruch 6 oder 7, worin das Akyl, dargestellt durch R³ bis R⁶ in der Formel (2), C₁ oder C₂ ist.

10. Nicht-medizinische Verwendung gemäß einem der Ansprüche 6 bis 9, worin der Gehalt der einen oder mehreren Verbindung, ausgewählt aus der Gruppe, bestehend aus Pentaerythrit, dem Alkylacetalderivat von Pentaerythrit mit der Formel (1) und dem Alkylacetalderivat von Pentaerythrit mit der Formel (2) von 0,001 bis 15 Masse-% auf der Basis der Gesamtmenge der Zusammensetzung ist.

## Revendications

1. Composition de traitement cutané comprenant un ou plusieurs composés, en tant que principe actif, sélectionnés dans le groupe consistant en le pentaérythritol, un dérivé alkylacétal de pentaérythritol représenté par la formule (1) décrite ci-dessous, et un dérivé alkylacétal de pentaérythritol représenté par la formule (2) décrite ci-dessous, pour son utilisation dans un procédé de suppression de la production de PGE₂ pour obtenir un effet anti-inflammatoire ou de suppression de la production de mélanine : 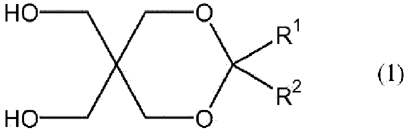 dans laquelle R¹ et R² peuvent être différents l'un de l'autre pour représenter un groupe alkyle en C₁₋₄ ; et 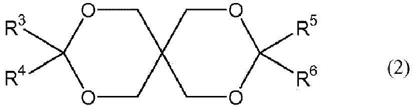 dans laquelle R³ à R⁶ peuvent être différents les uns des autres pour représenter un groupe alkyle en C₁₋₄.

2. Composition selon la revendication 1, dans laquelle le principe actif est le pentaérythritol.

3. Composition selon la revendication 1 ou 2, dans laquelle l'alkyle représenté par R¹ et R² dans la formule (1) est en C₁ ou en C₂.

4. Composition selon la revendication 1 ou 2, dans laquelle l'alkyle représenté par R³ à R⁶ dans la formule (2) est en C₁ ou en C₂.

5. Composition selon l'une quelconque des revendications 1 à 4, dans laquelle la teneur en les uns ou plusieurs composés sélectionnés dans le groupe consistant en le pentaérythritol, le dérivé alkylacétal de pentaérythritol représenté par la formule (1), et le dérivé alkylacétal de pentaérythritol représenté par la formule (2), est de 0,001 à 15 % en masse sur la base de la quantité totale de la composition.

6. Utilisation non médicale d'une composition de préparation externe pour la peau comprenant un ou plusieurs composés sélectionnés dans le groupe consistant en le pentaérythritol, un dérivé alkylacétal de pentaérythritol représenté par la formule (1) décrite ci-dessous, et un dérivé alkylacétal de pentaérythritol représenté par la formule (2) décrite ci-dessous, pour supprimer la production de mélanine : 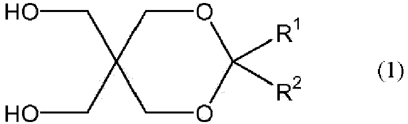 dans laquelle R¹ et R² peuvent être différents l'un de l'autre pour représenter un groupe alkyle en C₁₋₄ ; et 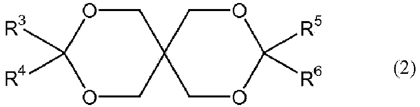 dans laquelle R³ à R⁶ peuvent être différents les uns des autres pour représenter un groupe alkyle en C₁₋₄.

7. Utilisation non médicale selon la revendication 6, dans laquelle le principe actif est le pentaérythritol.

8. Utilisation non médicale selon la revendication 6 ou 7, dans laquelle l'alkyle représenté par R¹ et R² dans la formule (1) est en C₁ ou en C₂.

9. Utilisation non médicale selon la revendication 6 ou 7, dans laquelle l'alkyle représenté par R³ à R⁶ dans la formule (2) est en C₁ ou en C₂.

10. Utilisation non médicale selon l'une quelconque des revendications 6 à 9, dans laquelle la teneur en les uns ou plusieurs composés sélectionnés dans le groupe consistant en le pentaérythritol, le dérivé alkylacétal de pentaérythritol représenté par la formule (1), et le dérivé alkylacétal de pentaérythritol représenté par la formule (2), est de 0,001 à 15 % en masse sur la base de la quantité totale de la composition.
